# EUROPEAN PATENT SPECIFICATION

(11) **EP 4 433 054 B1**
(45) Date of publication and mention of the grant of the patent: **08.07.2026**
(21) Application number: 22812750.2
(22) Date of filing: 18.11.2022
(51) Int. Cl.: A61K 31/47, A61K 31/4184, A61P 25/00, A61P 35/00

(54) **NITROXOLINE FOR USE IN THE TREATMENT OR PREVENTION OF A PLEXIFORM NEUROFIBROMA**
NITROXOLIN ZUR VERWENDUNG BEI DER BEHANDLUNG ODER VORBEUGUNG EINES PLEXIFORMEN NEUROFIBROMS
NITROXOLINE POUR SON UTILISATION DANS LE TRAITEMENT OU LA PRÉVENTION D'UN NEUROFIBROME PLEXIFORME

(30) Priority: 19.11.2021 GB 202116743
(43) Date of publication of application: 25.09.2024
(73) Proprietor: Healx Limited, Cambridge, Cambridgeshire CB2 1LA (GB)
(72) Inventor: BROWN, David, Cambridge CB2 1LA (GB)
(74) Representative: Potter Clarkson
(86) International application number: PCT/GB2022/052933
(87) International publication number: WO 2023/089328

(56) References cited:
- WO-A1-2022/258972
- WO-A2-2010/042163
- MITROVIC ANA ET AL: "Nitroxoline: repurposing its antimicrobial to antitumor application", ACTA BIOCHIMICA POLONICA, 13 December 2019 (2019-12-13), PL, pages 521 - 531, XP093021896, ISSN: 0001-527X, Retrieved from the Internet <URL:https://ojs.ptbioch.edu.pl/index.php/abp/article/download/2904/4183> DOI: 10.18388/abp.2019_2904
- ANONYMOUS: "MetAP2, A New Therapeutic Target in the NF1 Pathway", CANCER BIOLOGY & THERAPY, vol. 4, no. 12, 1 December 2005 (2005-12-01), US, pages 1301 - 1306, XP093021900, ISSN: 1538-4047, Retrieved from the Internet <URL:https://www.tandfonline.com/doi/epdf/10.4161/cbt.4.12.2325?needAccess=true&role=button> DOI: 10.4161/cbt.4.12.2325
- MUKHOPADHYAY SABYASACHI ET AL: "Selumetinib: the first ever approved drug for neurofibromatosis-1 related inoperable plexiform neurofibroma.", CURRENT MEDICAL RESEARCH AND OPINION, 1 May 2021 (2021-05-01), pages 789 - 794, XP093021878, Retrieved from the Internet <URL:http://www.ncbi.nlm.nih.gov/pubmed/33683166> [retrieved on 20230208]
- KILLOCK DAVID: "Selumetinib benefits children with inoperable plexiform neurofibromas", NATURE REVIEWS CLINICAL ONCOLOGY, NATURE, NY, US, vol. 17, no. 5, 31 March 2020 (2020-03-31), pages 273, XP037096162, ISSN: 1759-4774, [retrieved on 20200331], DOI: 10.1038/S41571-020-0361-7
- LAZOVIC JELENA ET AL: "Nitroxoline induces apoptosis and slows glioma growth in vivo", NEURO-ONCOLOGY, vol. 17, no. 1, 1 January 2015 (2015-01-01), US, pages 53 - 62, XP093021879, ISSN: 1522-8517, Retrieved from the Internet <URL:https://www.ncbi.nlm.nih.gov/pmc/articles/PMC4483047/pdf/nou139.pdf> DOI: 10.1093/neuonc/nou139
- KUMARI NISHA ET AL: "NITROXOLINE EXHIBIT ANTICANCER ACTIVITY INDUCING APOPTOSIS IN A TEMOZOLOMIDE-RESISTANT GLIOBLASTOMA", NEURO-ONCOLOGY, 1 November 2017 (2017-11-01), pages vi59, XP055950242

## Description

### Field of the invention

This invention relates to new uses of nitroxoline, in particular to nitroxoline, or a pharmaceutically acceptable salt thereof, for use in the treatment or prevention of a plexiform neurofibroma.

### Background of the invention

A neurofibroma is a benign nerve-sheath tumour in the peripheral nervous system. In 90% of cases, they are found as stand-alone tumours, while the remainder are found in persons with neurofibromatosis type I (NF1), an autosomal-dominant genetically inherited disease. Neurofibromas can result in a range of symptoms from physical disfiguration and pain to cognitive disability and can transform into malignant tumours.

Plexiform neurofibromas arise during early development from nerves in the skin, or from more internal nerve bundles such as cranial nerves or proximal large peripheral nerve sheaths. Plexiform neurofibromas are composed of Schwann cells (SC), fibroblasts, degranulating mast cells, and vascular cells (Hirota S et al., Arch Pathol Lab Med. 1993;117(10):996-9). Plexiform neurofibromas can expand progressively and constitute a lifelong source of morbidity and mortality, with a 10-15% lifetime incidence of transformation to malignant peripheral nerve sheath tumor.

NF1 is caused by germline mutations of the *NF1* tumor suppressor gene, which encodes the protein neurofibromin. Neurofibromin functions as a GTPase-activating (GAP) protein and inactivates the intracellular signal transduction protein Ras by converting the active GTP-bound form into its inactive GDP-bound form. This in turn leads to the downregulation of Ras activity. Loss of neurofibromin activity increases Ras activity, which in turn promotes the transcription of a number of genes required for cell growth and proliferation. Plexiform neurofibromas appear in approximately 15% to 40% of patients with NF1.

Internal plexiform neurofibromas are very difficult to remove completely by surgery because they extend through multiple layers of tissue and the attempt would damage healthy tissue or organs. Plexiform neurofibroma can cause disfigurement, neurological, and other clinical deficits including having the potential to cause severe clinical complications if they occur in certain areas.

There have also been considerable efforts to identify pharmacological targets to treat plexiform neurofibromas. In particular plexiform neurofibromas have been a frequent target of repurposing efforts as well as repositioning of drugs in development. Many different standards and methods have been applied to this task. In many cases, repurposing candidates have been identified based primarily on clinical pattern matching, while in others basic disease mechanisms have been studied extensively to identify therapeutic targets, followed by thorough preclinical validation.

At present, surgery remains the primary treatment option. However, removal is difficult because they can be large and cross tissue boundaries. In 2020, the FDA has approved the MEK inhibitor Koselugo (selumetinib) for the treatment of paediatric patients two years of age and older with NF1 who have symptomatic, inoperable plexiform neurofibromas. However, not all patients respond to the treatment and tumors only shrink partially. Selumetinib is a selective inhibitor of mitogen-activated protein kinase kinase (MAPK kinase, MEK, MAP2K, and MAPKK) and has the systemic name 6-(4-bromo-2-chloroanilino)-7-fluoro-N-(2-hydroxyethoxy)-3-methylbenzimidazole-5-carboxamide.

Overall, efforts to treat plexiform neurofibromas have led to some exciting possibilities, but no definitive successes, despite much effort. This has highlighted the need for new therapies.

Nitroxoline is used in humans as an antibiotic, it is not widely used but has been on the market since the 1960s. It is used in the treatment or prevention of biofilm infections, such as urinary tract infections. It is particularly effective at disrupting biofilms and it is the metal cation chelation property that is believed to be responsible for this action. Nitroxoline is metabolised in the liver to the corresponding sulphate and glucuronide metabolites. There is evidence that the metabolites both share the antimicrobial activity. It has also been used in anticancer settings via antiproliferative action. Nitroxoline has the systematic name 5-nitroquinolin-8-ol.

WO 2010/042163 discloses that nitroxoline is a selective inhibitor of MetAP2 and SIRT1 and can be used for the treatment of diseases associated therewith.

MetAP2, A New Therapeutic Target in the NF1 Pathway (Cancer Biology & Therapy, vol. 4, no. 12, 1 December 2005, pages 1301-1306) discloses targeting MetAP2 for the treatment of neurofibromatosis 1.

### Summary of the invention

The references to methods of treatment in this description are to be interpreted as references to compounds, pharmaceutical compositions and medicaments of the present invention for use in a method for treatment of the human (or animal) body by therapy.

The present invention is a composition comprising nitroxoline, or a pharmaceutically acceptable salt thereof, for use in the treatment or prevention of a plexiform neurofibroma. As will be evident from the *in vitro* data presented below, nitroxoline is effective in treating and preventing a plexiform neurofibroma.

A first aspect of the invention is a composition comprising nitroxoline, or a pharmaceutically acceptable salt thereof, for use in the treatment or prevention of a plexiform neurofibroma.

### Description of the figures

Figure 1 shows the nitroxoline dose-response in proliferation (top) and apoptosis (bottom) of WT and *NF1* deficient Schwann cells in *in vitro* assays.
Figure 2 shows the effect of nitroxoline treatment on proximal nerve volume (A) and tumour number (B) in the *Postn-*Cre*⁺ Nf1*^{*fl*/*fl*} mouse model of plexiform neurofibroma (n=6, * = p value <0.05 Fisher's LSD test/ Dunnett's test).
Figure 3 shows a graphic of the tumour/nerve width and how the caliper measurement is taken.

### Detailed description

Non-myelinating Schwann cells that only express the inactive version of the *NF1* gene (a "tumour suppressor gene") leading to a complete loss of expression of functional neurofibromin are the origin of plexiform neurofibromas. The *NF1* gene codes for a protein that regulates cell growth. There are two kinds of Schwann cells, myelinating and non-myelinating. While myelinating Schwann cells cover large diameter (>1 micrometer) peripheral nervous system (PNS) axons with myelin, non-myelinating Schwann cells encapsulate small diameter PNS axons with their cytoplasmic processes. In non-mutated non-myelinating Schwann cells, their conglomeration around axons is called a Remak bundle. Whereas, mutated non-myelinating Schwann cells do not form normal Remak bundles. Instead, they fail to properly surround and segregate target axons causing the plexiform neurofibromas. Furthermore, once a non-myelinating Schwann cell has suffered inactivation of its *NF1* genes, it begins to proliferate rapidly.

It has been hypothesized that the proliferating non-myelinating Schwann cells secrete chemoattractants that promote the migration of different kinds of cells that are heterozygous for the *NF1* gene into the hyperplastic lesions created by the non-myelinating Schwann cells. These cell types include fibroblasts, perineurial cells, endothelial cells, and mast cells. The mast cells then secrete mitogens or survival factors that alter the developing tumor microenvironment and result in neurofibroma formation.

In the present invention, and as demonstrated by the below *in vitro* data, nitroxoline inhibits cell proliferation and increases apoptosis in *NF1* deficient Schwann cells, and is therefore an effective treatment of a plexiform neurofibroma. Preferably, nitroxoline is used for the treatment or prevention of a plexiform neurofibroma, wherein the subject has neurofibromatosis type I.

By the term "treatment" or "treating" as used herein, we refer to therapeutic (curative) treatment, which includes reducing the size of a plexiform neurofibroma. A blood test for protein melanoma inhibitory activity may be used to detect the presence of neurofibromas. By the term "prevention" or "preventing" as used herein, we refer to "prophylactic" treatment, which includes administering the compositions of the invention to a patient that has mutated (e.g. *NF1* deficient) non-myelinating Schwann cells but a plexiform neurofibroma has not developed. The mutated (e.g. *NF1* deficient) non-myelinating Schwann cells may have begun to proliferate, such as to proliferate rapidly.

"Patient" and "subject" are used interchangeably and refer to the subject that is to be administered the nitroxoline. Preferably the subject is a human. Suitably the subject has neurofibromatosis type I. In one embodiment the patient is a paediatric patient, preferably a paediatric patient 2 years of age and older, preferably the paediatric patient has NF1.

In one embodiment, nitroxoline is used for the treatment or prevention of a plexiform neurofibroma, wherein the patient has had or is going to have surgery to remove some or all of the plexiform neurofibroma. This may be particularly advantageous if the plexiform neurofibroma is large and/or expands across tissue boundaries, so it is difficult to remove it all by surgery and/or a quick removal of at least some of it is desired/beneficial.

The term "surgery" has its normal meaning in the art. Surgery is an invasive technique with the fundamental principle of physical intervention on organs/organ systems/tissues for diagnostic or therapeutic reasons.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as citric, fumaric, maleic, malic, ascorbic, succinic, tartaric, benzoic, acetic, methanesulfonic, ethanesulfonic, salicylic, stearic, benzenesulfonic or *p*-toluenesulfonic acid. Pharmaceutically acceptable bases include alkali metal (e.g. sodium or potassium) and alkali earth metal (e.g. calcium or magnesium) hydroxides and organic bases such as alkyl amines, aryl amines or heterocyclic amines.

The present invention is directed to a composition comprising nitroxoline, or a pharmaceutically acceptable salt thereof, for use in the treatment or prevention of a plexiform neurofibroma.

In an alternative embodiment, the present invention is directed to a composition comprising nitroxoline, or a pharmaceutically acceptable salt thereof, for use in the treatment or prevention of a plexiform neurofibroma, wherein nitroxoline is the only active agent in the composition. By only active agent it is meant that the composition does not contain other components which may be used in the treatment or prevention of a plexiform neurofibroma. In an alternative embodiment, the composition further comprises a second active agent for treating plexiform neurofibroma, preferably wherein the second active agent is selumetinib, or a pharmaceutically acceptable salt thereof.

In an alternative embodiment, the present invention is directed to a composition comprising nitroxoline, or a pharmaceutically acceptable salt thereof, for use in combination with a second composition comprising selumetinib, or a pharmaceutically acceptable salt thereof, wherein the two compositions are administered to the subject simultaneously, separately or sequentially.

As used herein, "separate" administration means that the drugs are administered as part of the same overall dosage regimen (which could comprise a number of days), but preferably on the same day. As used herein "simultaneously" means that the drugs are to be taken together or formulated as a single composition. As used herein, "sequentially" means that the drugs are administered at about the same time, and preferably within about 1 hour of each other. Preferably, the drugs are administered simultaneously i.e. taken together or formulated as a single composition. Most preferably, they are formulated as a single composition.

The compositions of the invention may contain a pharmaceutically acceptable carrier. By "pharmaceutically acceptable carrier" is meant any diluent or excipient, such as fillers or binders, that is compatible with the other ingredients of the composition, and which is not deleterious to the recipient. The pharmaceutically acceptable carrier can be selected on the basis of the desired route of administration, in accordance with standard pharmaceutical practices.

In the present invention, the composition may be administered in a variety of dosage forms. In one embodiment, the composition may be formulated in a format suitable for oral, rectal, parenteral, intranasal or transdermal administration or administration by inhalation or by suppository.

The composition may be administered orally, for example as tablets, troches, lozenges, aqueous or oily suspensions, dispersible powders or granules. Preferably, the composition is formulated such that it is suitable for oral administration, for example tablets and capsules. Tablets and capsules may be prepared with binding agents, for example, syrup, acacia, gelatin, sorbitol, tragacanth, celluloses or polyvinylpyrrolidone; fillers, such as lactose, sucrose, corn starch, calcium phosphate, sorbitol, or glycine; lubricants, such as magnesium stearate, talc, polyethylene glycol, or silica; and surfactants, such as sodium lauryl sulfate. Liquid compositions may contain conventional additives such as suspending agents, for example sorbitol syrup, methyl cellulose, sugar syrup, gelatin, carboxymethyl-cellulose, or edible fats; emulsifying agents and surfactants such as lecithin, or acacia; vegetable oils such as almond oil, coconut oil, cod liver oil, or peanut oil; preservatives such as butylated hydroxyanisole (BHA) and butylated hydroxytoluene (BHT). Liquid compositions may be encapsulated in, for example, gelatin to provide a unit dosage form.

The composition may also be administered parenterally, whether subcutaneously, intravenously, intramuscularly, intrasternally, transdermally or by infusion techniques.

The composition may also be administered by inhalation. An advantage of inhaled medications is their direct delivery to the area of rich blood supply in comparison to many medications taken by oral route. Thus, the absorption is very rapid as the alveoli have an enormous surface area and rich blood supply and first pass metabolism is bypassed.

The present disclosure also provides an inhalation device containing the composition of the present invention. Typically said device is a metered dose inhaler (MDI), which contains a pharmaceutically acceptable chemical propellant to push the medication out of the inhaler.

The composition may also be administered by intranasal administration. The nasal cavity's highly permeable tissue is very receptive to medication and absorbs it quickly and efficiently. Nasal drug delivery is less painful and invasive than injections, generating less anxiety among patients. By this method absorption is very rapid and first pass metabolism is usually bypassed, thus reducing inter-patient variability. Further, the present disclosure also provides an intranasal device containing the composition according to the present invention.

The composition may also be administered by transdermal administration. For topical delivery, transdermal and transmucosal patches, creams, ointments, jellies, solutions or suspensions may be employed. The present disclosure therefore also provides a transdermal patch containing the composition.

The composition may also be administered by sublingual administration. The present disclosure therefore also provides a sub-lingual tablet comprising the composition.

The composition may also be formulated with an agent which reduces degradation of the substance by processes other than the normal metabolism of the patient, such as anti-bacterial agents, or inhibitors of protease enzymes which might be the present in the patient or in commensural or parasite organisms living on or within the patient, and which are capable of degrading the compound.

Liquid dispersions for oral administration may be syrups, emulsions and suspensions.

Suspensions and emulsions may contain as carrier, for example a natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for injection or infusion may contain as carrier, for example, sterile water or preferably they may be in the form of sterile, aqueous, isotonic saline solutions.

In an embodiment of the invention, the composition is administered in an effective amount to treat or prevent a plexiform neurofibroma. An effective dose will be apparent to one skilled in the art, and is dependent on a number of factors including age, sex, weigh, which the medical practitioner will be capable of determining.

In a preferred embodiment, the composition comprises 30 mg to 600 mg, preferably 50 mg to 500 mg, more preferably 100 mg to 400 mg, yet more preferably 150 mg to 350 mg, most preferably 200 mg to 300 mg nitroxoline.

The composition may be administered once a day, twice a day, three times a day or four times a day.

In an embodiment of the invention, the composition is administered at least once a day. Preferably it is administered as a single daily dose. Preferably the single daily dose is 90 mg to 1800 mg, preferably 150 mg to 1500 mg, more preferably 300 mg to 1200 mg, yet more preferably 450 mg to 1050 mg, most preferably 600 mg to 900 mg of nitroxoline.

In an embodiment of the invention, the composition is administered twice daily. Preferably each dose is 45 mg to 900 mg, preferably 75 mg to 750 mg, more preferably 150 mg to 600 mg, yet more preferably 225 mg to 525 mg, most preferably 300 mg to 450 mg of nitroxoline.

In an embodiment of the invention, the composition is administered three times daily. Preferably each dose is 30 mg to 600 mg, preferably 50 mg to 500 mg, more preferably 100 mg to 400 mg, yet more preferably 150 mg to 350 mg, most preferably 200 mg to 300 mg of nitroxoline.

In an embodiment of the invention, the composition is administered four times daily. Preferably each dose is 15 mg to 500 mg, preferably 50 mg to 400 mg, more preferably 100 mg to 300 mg, yet more preferably 125 mg to 225 mg, most preferably 150 mg to 200 mg of nitroxoline.

Preferably, the dosage regime is such that the total daily dosage of nitroxoline does not exceed 1500 mg.

Suitably the dosage of nitroxoline may be between 50 and 250 mg/kg, more preferably between 60 and 200 mg/kg, even more preferably between 80 and 170 mg/kg, such as between 100 and 150 mg/kg.

Suitably the effective dose of nitroxoline results in a concentration of 1 to 150 µM, preferably 10 to 100 µM, more preferably 25 to 50 µM in cells.

Suitably the composition comprising nitroxoline and the second composition comprising the second active agent, preferably selumetinib, are a single daily dose. Suitably the two compositions are administered simultaneously i.e. nitroxoline and selumetinib are taken together. The compositions may also be administered sequentially i.e. at about the same time, and preferably within about 1 hour of each other.

In the embodiments wherein the composition comprises selumetinib or the composition is for use in combination with a second composition comprising selumetinib, suitably the compositions comprising selumetinib comprise between 1 mg and 75 mg of selumetinib, preferably between 5 mg to 50 mg of selumetinib, more preferably between 10 mg to 35 mg of selumetinib, most preferably between 15 mg to 30 mg of selumetinib.

Suitably the effective dose of selumetinib administered to the subject is between 1 mg/m² and 75 mg/m² of selumetinib, preferably between 5 mg/m² to 50 mg/m² of selumetinib, more preferably between 10 mg/m² to 35 mg/m² of selumetinib, most preferably between 15 mg/m² to 30 mg/m² of selumetinib.

In order to treat or prevent a plexiform neurofibroma, the composition comprising nitroxoline is used in a chronic dosage regime i.e. chronic, long-term treatment. Suitably the regime lasts for at least one month, suitably at least two months, such as at least three months.

The present disclosure also relates to a kit comprising: (i) at least one dose of nitroxoline, or a pharmaceutically acceptable salt thereof; and optionally (ii) at least one dose of selumetinib, or a pharmaceutically acceptable salt thereof, for simultaneous, separate or sequential use in the treatment or prevention of a plexiform neurofibroma.

Also disclosed (but not part of the claimed invention) are prodrugs which react *in vivo* to give a compound of the present invention.

### Experimental Section

### Example 1 - In vitro drug testing utilizing WT and NF1 deficient Schwann cells

In this study the efficacy of nitroxoline was investigated and its ability to reduce cellular proliferation, increase apoptosis, and overall cell viability utilizing immortalized *wild-type* (*WT*) and *NF1* deficient (*Nf1* ^{-/-}) Schwann cells (SCs).

*Nf1* ^{-/-} SCs have increased survival and proliferation *in vitro,* consistent with the *in vivo* phenotypes (Kim HA et al., Mol Cell Biol. 1997;17(2):862-72). The present studies utilize *WT* (ipn02.3A) and *NF1* ^{-/-} (ipNF95.6) immortalized human-derived SC lines. The selectivity of nitroxoline was compared between *NF1* ^{-/-} and *WT* cells.

Cells were treated with serially diluted nitroxoline starting from 105 µM and incubated for 48 hours. After the incubation period, proliferation, viability, and apoptosis assays were performed as described below:
- Cell proliferation was assessed using the CellTiter-Glo Assay (Promega) which measures ATP consumption. Briefly, *WT* and *Nf1* ^{-/-} cells were plated in triplicate at a concentration of 10,000 cells/well in 96-well dishes in 100 µl DMEM containing 1% glutamine, 10% FBS, 2% Sodium Bicarbonate and 1% Penicillin/Streptomycin, in a 37°C, 5% CO2 humidified incubator with or without the addition of nitroxoline for 48 hours. Following incubation, 100µl of CellTiterGlo reagent was added to each well. After 10minutes, the plates were read using a 96-well microplate luminometer.
- **Cellular apoptosis** was assessed using the Caspase-Glo 3/7 kit (Promega). Briefly, *WT* and *Nf1* ^{-/-} cells were plated in triplicate at a concentration of 10,000 cells/well in 96-well dishes in 100 µl DMEM containing 1% glutamine, 10% FBS, 2% Sodium Bicarbonate and 1% Penicillin/Streptomycin, in a 37°C, 5% CO2 humidified incubator with or without the addition of nitroxoline for 48 hours. Following incubation, 100µl Caspase-Glo 3/7 reagent was added to each well. After 10 minutes, plates were read using a 96 well luminometer and caspase 3/7 activity was measured.

### Results

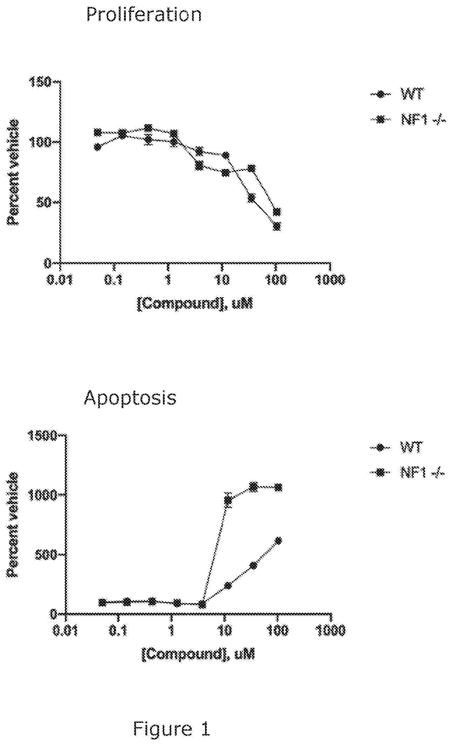

Nitroxoline inhibited cell proliferation and increased apoptosis in a dose-response manner, as seen in Figure 1. The antiproliferative effect was observed at concentrations greater than 1 µM in *WT* and *Nf1* -/- Schwann cells. The induction of apoptosis was evident at concentrations greater than 3 µM in both SCs but the magnitude of the increase was significantly greater in *Nf1* -/- SC at concentrations greater than 10 µM.

### Example 2 - In vivo drug testing in Nf1-KO mice

The aim of this study was to evaluate the effect of nitroxoline in a mouse model of neurofibromatosis type 1, more specifically a model that develops plexiform neurofibromas. This study is the same used by AstraZeneca for the approval of selumetinib for pNF.

### Animals

This study uses the *Postn-*Cre⁺ *Nf1*^{*fl*/*fl*} mouse model of plexiform neurofibroma. The neurofibromas that arise in these mice faithfully recapitulate human disease (Burks et al. 2019). At 4 months of age when mice (male and female) had established plexiform neurofibromas they were randomised into three treatment groups and administered nitroxoline at 120mg/kg QD, 120mg/kg BD or vehicle (10% DMSO in 90% corn oil) via oral gavage. Mice were treated for 12 weeks then sacrificed. Mice were perfused and fixed in 10% neutral buffered formalin.

### Nerve volume

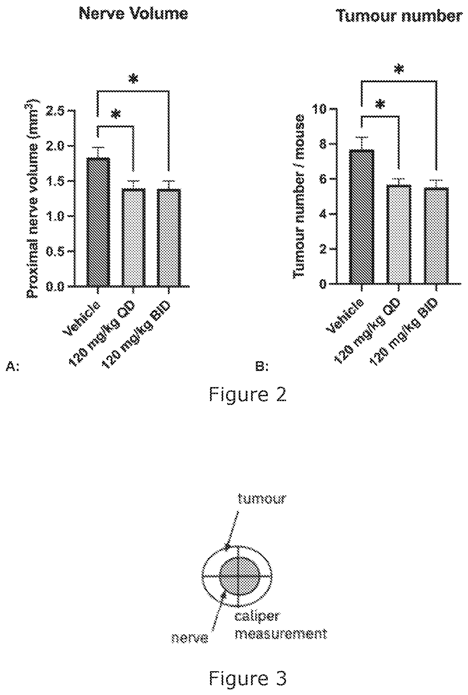

The whole bodies were decalcified in 5% formic acid, the spinal proximal nerve tree was dissected, and nerve width (including the tumour formed along it) measured via calliper (shown in Figure 3) across four nerves per mouse. Nerve volume was subsequently calculated using a spheroid calculation: 0.52 x (width)2 w length.

### Tumour number

To quantify tumour number, the nerves were processed to paraffin blocks, sectioned, and stained for collagen using Masson's trichrome stain. Tumour number was scored from these slides by a clinician.

### Statistical Analysis

Graphs and statistical analysis of data were produced using GraphPad Prism (Ver 9).

### Results

Mice treated with nitroxoline at both dose schedules (120mg/kg QD and 120mg/kg BD) had lower proximal nerve volumes compared to vehicle treated control mice (Figure 2). Plexiform tumours form along the proximal nerves, therefore a reduction in proximal nerve volume can be interpreted as a reduction in plexiform tumour size. In addition to a reduction in total nerve volume, a reduction in tumour number along the nerves was observed in nitroxoline treated mice compared to vehicle controls (Figure 2).

### Conclusions

Nitroxoline inhibits cell proliferation and increases apoptosis *in vitro* in Nf1 -/-Schwann cells. In a mouse model of plexiform neurofibroma, nitroxoline treated mice had lower proximal nerve volumes and less tumours compared to vehicle control animals. It is thus expected that nitroxoline will reduce, treat and prevent plexiform neurofibromas.

### References

Burks CA, Rhodes SD, Bessler WK, Chen S, Smith A, Gehlhausen JR, Hawley ET, Jiang L, Li X, Yuan J, Lu Q, Jacobsen M, Sandusky GE, Jones DR, Clapp DW, Blakeley JO. Ketotifen Modulates Mast Cell Chemotaxis to Kit-Ligand, but Does Not Impact Mast Cell Numbers, Degranulation, or Tumor Behavior in Neurofibromas of Nf1-Deficient Mice. Mol Cancer Ther. 2019 Dec;18(12):2321-2330.

## Claims

1. A composition comprising nitroxoline, or a pharmaceutically acceptable salt thereof, for use in the treatment or prevention of a plexiform neurofibroma.

2. The composition for use according to claim 1 in the treatment of a plexiform neurofibroma.

3. The composition for use according to either claim 1 or 2 wherein the subject of the treatment or prevention has neurofibromatosis type I.

4. The composition for use according to any preceding claim, wherein the subject of the treatment or prevention is human.

5. The composition for use according to any preceding claim, wherein the composition comprises 30 mg to 600 mg, preferably 50 mg to 500 mg, more preferably 100 mg to 400 mg, yet more preferably 150 mg to 350 mg, most preferably 200 mg to 300 mg of nitroxoline.

6. The composition for use according to any preceding claim, wherein administration is by a dose two times per day.

7. The composition for use according to claim 6, wherein the dose is 45 mg to 900 mg, preferably 75 mg to 750 mg, more preferably 150 mg to 600 mg, yet more preferably 225 mg to 525 mg, most preferably 300 mg to 450 mg of nitroxoline.

8. The composition for use according to any of claims 1 to 4, where administration is by a dose three times per day and wherein the dose is 30 mg to 600 mg, preferably 50 mg to 500 mg, more preferably 100 mg to 400 mg, yet more preferably 150 mg to 350 mg, most preferably 200 mg to 300 mg of nitroxoline.

9. The composition for use according to any of claims 1 to 4, where administration is by a dose four times per day and wherein the dose is 15 mg to 500 mg, preferably 50 mg to 400 mg, more preferably 100 mg to 300 mg, yet more preferably 125 mg to 225 mg, most preferably 150 mg to 200 mg of nitroxoline.

10. The composition for use according to any preceding claim, to be administered orally or intravenously.

11. The composition for use according to any of claims 1 to 9, to be administered by parenteral, transdermal, sublingual, rectal or inhaled administration.

12. The composition for use according to any preceding claim wherein nitroxoline, or the pharmaceutically acceptable salt, is the only active agent in the composition.

13. The composition for use according to any one of claims 1 to 11, wherein the composition further comprises selumetinib, or a pharmaceutically acceptable salt thereof.

14. The composition for use according to any one of claims 1 to 12, for use in combination with a second composition comprising selumetinib, or a pharmaceutically acceptable salt thereof, wherein the two compositions are administered to the subject simultaneously, separately or sequentially.

15. The composition for use according to claims 13 or 14 wherein the amount of selumetinib is between 1 mg and 75 mg, preferably between 5 mg to 50 mg, more preferably between 10 mg to 35 mg, most preferably between 15 mg to 30 mg.

## Patentansprüche

1. Zusammensetzung, umfassend Nitroxolin oder ein pharmazeutisch verträgliches Salz davon, zur Verwendung bei der Behandlung oder Vorbeugung eines plexiformen Neurofibroms.

2. Zusammensetzung zur Verwendung nach Anspruch 1 bei der Behandlung eines plexiformen Neurofibroms.

3. Zusammensetzung zur Verwendung nach Anspruch 1 oder 2, wobei das Subjekt der Behandlung oder Vorbeugung Neurofibromatose Typ I aufweist.

4. Zusammensetzung zur Verwendung nach einem der vorstehenden Ansprüche, wobei das Subjekt der Behandlung oder Vorbeugung ein Mensch ist.

5. Zusammensetzung zur Verwendung nach einem der vorstehenden Ansprüche, wobei die Zusammensetzung 30 mg bis 600 mg, vorzugsweise 50 mg bis 500 mg, mehr bevorzugt 100 mg bis 400 mg, noch mehr bevorzugt 150 mg bis 350 mg, am meisten bevorzugt 200 mg bis 300 mg, Nitroxolin umfasst.

6. Zusammensetzung zur Verwendung nach einem der vorstehenden Ansprüche, wobei eine Verabreichung durch eine Dosis zweimal täglich erfolgt.

7. Zusammensetzung zur Verwendung nach Anspruch 6, wobei die Dosis 45 mg bis 900 mg, vorzugsweise 75 mg bis 750 mg, mehr bevorzugt 150 mg bis 600 mg, noch mehr bevorzugt 225 mg bis 525 mg, am meisten bevorzugt 300 mg bis 450 mg, Nitroxolin beträgt.

8. Zusammensetzung zur Verwendung nach einem der Ansprüche 1 bis 4, wobei die Verabreichung durch eine Dosis dreimal täglich erfolgt, und wobei die Dosis 30 mg bis 600 mg, vorzugsweise 50 mg bis 500 mg, mehr bevorzugt 100 mg bis 400 mg, noch mehr bevorzugt 150 mg bis 350 mg, am meisten bevorzugt 200 mg bis 300 mg, Nitroxolin beträgt.

9. Zusammensetzung zur Verwendung nach einem der Ansprüche 1 bis 4, wobei die Verabreichung durch eine Dosis viermal täglich erfolgt, und wobei die Dosis 15 mg bis 500 mg, vorzugsweise 50 mg bis 400 mg, mehr bevorzugt 100 mg bis 300 mg, noch mehr bevorzugt 125 mg bis 225 mg, am meisten bevorzugt 150 mg bis 200 mg, Nitroxolin beträgt.

10. Zusammensetzung zur Verwendung nach einem der vorstehenden Ansprüche, die oral oder intravenös zu verabreichen ist.

11. Zusammensetzung zur Verwendung nach einem der Ansprüche 1 bis 9, die durch parenterale, transdermale, sublinguale, rektale oder inhalierte Verabreichung zu verabreichen ist.

12. Zusammensetzung zur Verwendung nach einem der vorstehenden Ansprüche, wobei Nitroxolin oder das pharmazeutisch verträgliche Salz der einzige Wirkstoff in der Zusammensetzung ist.

13. Zusammensetzung zur Verwendung nach einem der Ansprüche 1 bis 11, wobei die Zusammensetzung ferner Selumetinib oder ein pharmazeutisch verträgliches Salz davon umfasst.

14. Zusammensetzung zur Verwendung nach einem der Ansprüche 1 bis 12, zur Verwendung in Kombination mit einer zweiten Zusammensetzung, umfassend Selumetinib oder ein pharmazeutisch verträgliches Salz davon, wobei die zwei Zusammensetzungen dem Subjekt gleichzeitig, getrennt oder nacheinander verabreicht werden.

15. Zusammensetzung zur Verwendung nach dem Anspruch 13 oder 14, wobei die Menge an Selumetinib zwischen 1 mg und 75 mg, vorzugsweise zwischen 5 mg und 50 mg, mehr bevorzugt zwischen 10 mg und 35 mg, am meisten bevorzugt zwischen 15 mg und 30 mg, beträgt.

## Revendications

1. Composition comprenant de la nitroxoline, ou un sel pharmaceutiquement acceptable de celle-ci, pour une utilisation dans le traitement ou la prévention d'un neurofibrome plexiforme.

2. Composition pour une utilisation selon la revendication 1, pour le traitement d'un neurofibrome plexiforme.

3. Composition pour une utilisation selon la revendication 1 ou 2, dans laquelle le sujet du traitement ou de la prévention souffre d'une neurofibromatose de type I.

4. Composition pour une utilisation selon l'une quelconque revendication précédente, dans laquelle le sujet du traitement ou de la prévention est humain.

5. Composition pour une utilisation selon l'une quelconque revendication précédente, dans laquelle la composition comprend 30 mg à 600 mg, de préférence 50 mg à 500 mg, plus préférablement 100 mg à 400 mg, encore plus préférablement 150 mg à 350 mg, le plus préférablement 200 mg à 300 mg, de nitroxoline.

6. Composition pour une utilisation selon l'une quelconque revendication précédente, dans laquelle l'administration se fait par une dose deux fois par jour.

7. Composition pour une utilisation selon la revendication 6, dans laquelle la dose comprend 45 mg à 900 mg, de préférence 75 mg à 750 mg, plus préférablement 150 mg à 600 mg, encore plus préférablement 225 mg à 525 mg, le plus préférablement 300 mg à 450 mg, de nitroxoline.

8. Composition pour une utilisation selon l'une quelconque des revendications 1 à 4, l'administration se faisant par une dose trois fois par jour et dans laquelle la dose comprend 30 mg à 600 mg, de préférence 50 mg à 500 mg, plus préférablement 100 mg à 400 mg, encore plus préférablement 150 mg à 350 mg, le plus préférablement 200 mg à 300 mg, de nitroxoline.

9. Composition pour une utilisation selon l'une quelconque des revendications 1 à 4, l'administration se faisant par une dose quatre fois par jour et dans laquelle la dose comprend 15 mg à 500 mg, de préférence 50 mg à 400 mg, plus préférablement 100 mg à 300 mg, encore plus préférablement 125 mg à 225 mg, le plus préférablement 150 mg à 200 mg, de nitroxoline.

10. Composition pour une utilisation selon l'une quelconque revendication précédente, à administrer par voie orale ou par voie intraveineuse.

11. Composition pour une utilisation selon l'une quelconque des revendications 1 à 9, à administrer par administration par voie parentérale, transdermique, sublinguale, rectale ou par inhalation.

12. Composition pour une utilisation selon l'une quelconque revendication précédente, dans laquelle la nitroxoline, ou le sel pharmaceutiquement acceptable, est la seule substance active dans la composition.

13. Composition pour une utilisation selon l'une quelconque des revendications 1 à 11, dans laquelle la composition comprend en outre du sélumétinib, ou un sel pharmaceutiquement acceptable de celui-ci.

14. Composition pour une utilisation selon l'une quelconque des revendications 1 à 12, pour une utilisation en combinaison avec une seconde composition comprenant du sélumétinib, ou un sel pharmaceutiquement acceptable de celui-ci, dans laquelle les deux compositions sont administrées au sujet simultanément, séparément ou séquentiellement.

15. Composition pour une utilisation selon la revendication 13 ou 14, dans laquelle la quantité de sélumétinib est comprise entre 1 mg et 75 mg, de préférence entre 5 mg et 50 mg, plus préférablement entre 10 mg et 35 mg, le plus préférablement entre 15 mg et 30 mg.
